(19) Europäisches Patentamt
European Patent Office
Office européen des brevets

(11) EP 3 643 244 A1

(12) EUROPEAN PATENT APPLICATION

(43) Date of publication:
29.04.2020 Bulletin 2020/18

(51) Int Cl.:
A61B 8/06 $^{(2006.01)}$     A61B 8/12 $^{(2006.01)}$
A61B 5/0215 $^{(2006.01)}$     A61B 5/0285 $^{(2006.01)}$
A61B 5/02 $^{(2006.01)}$     A61B 5/00 $^{(2006.01)}$
A61B 5/026 $^{(2006.01)}$     A61B 5/0456 $^{(2006.01)}$

(21) Application number: 18202488.5

(22) Date of filing: 25.10.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• MUELLER, Manfred
5656 AE Eindhoven (NL)

• VAN DER HORST, Arjen
5656 AE Eindhoven (NL)
• HAARTSEN, Jacob Roger
5656 AE Eindhoven (NL)
• FRACKOWIAK, Bruno Jean François
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) SYSTEM AND METHOD FOR DETERMINING PULSE WAVE VELOCITY OF VESSELS

(57) The invention is directed to systems and methods for determining a fundamental pulse wave velocity value for a vessel with minimized effect of reflections originating from the vessel network connected distal to the respective vessel.

Fig. 3

EP 3 643 244 A1

**Description**

FIELD OF THE INVENTION

The present invention is directed to a system and method for determining a value of a pulse-wave velocity in a region of a blood vessel.

BACKGROUND OF THE INVENTION

Hypertension and its associated conditions, chronic heart failure (CHF) and chronic renal failure (CRF), constitute a significant and growing global health concern. Current therapies for these conditions span the gamut covering non-pharmacological, pharmacological, surgical, and implanted device-based approaches. Despite the vast array of therapeutic options, the control of blood pressure and the efforts to prevent the progression of heart failure and chronic kidney disease remain unsatisfactory.

Blood pressure is controlled by a complex interaction of electrical, mechanical, and hormonal forces in the body. The main electrical component of blood pressure control is the sympathetic nervous system, a part of the body's autonomic nervous system, which operates without conscious control. The sympathetic nervous system connects the brain, the heart, the kidneys, and the peripheral blood vessels, each of which plays an important role in the regulation of the body's blood pressure.

Renal denervation is a treatment option for resistant hypertension. However, the efficacy of renal denervation may be very variable between patients. Recent studies (Finegold et al., Systematic evaluation of haemodynamic parameters to predict haemodynamic responders to renal artery denervation, EuroIntervention, Abstracts EuroPCR 2016) indicate that the pulse wave velocity (PWV) inside the main renal artery may be indicative of the outcome of renal denervation.

US 2010/0113949 A1 describes systems and methods that enable measurement of the velocity of a pulse wave propagating within a body lumen such as a blood vessel using a pulse-wave velocity determining device such as an intravascular elongate medical device. The elongate medical device can include a data collection device configured to collect pulse wave data at a location within the lumen. The data collection device is communicatively coupled with a velocity measurement system and configured to output the collected data to the velocity measurement system for determining a value of a pulse-wave velocity. The velocity measurement system is configured to calculate the velocity of the pulse wave based on the collection data.

SUMMARY OF THE INVENTION

It is an object of the present invention to improve pulse wave velocity measurements for short vessels.

According to a first aspect of the present invention, an apparatus is provided, comprising:

an input unit configured to receive a plurality of measurements along a length of a vessel of a patient;
a processor configured to:

ascertain a plurality of pulse wave velocity values along the length of the vessel based on the plurality of measurements;
ascertain a limit value of the pulse wave velocity values for classification of the patient according to a predicted therapeutic benefit.

PWV can be derived from measurements of various parameters obtained within or from outside of a vessel. For long vessels such as major arteries like the aorta, measuring the PWV is relatively easy. The length of the vessel allows measurement of the time delay necessary for the propagation of the pulse wave over a large distance. The measured PWV value is usually a single value, e.g. spatial average over a relatively long distance.

The inventors arrived to the insight that the measured PWV can vary along the length of even a homogenous vessel due to the effect of wave reflections from distal vessel network, e.g. bifurcations, stenoses or other structures causing non-ideal impedance matching. These reflected waves influence the measured PWV. Typically, these deviations from the "fundamental" PWV (PWV due to the compliance of the vessel without effect of reflection) depend on the distance of the measurement from the structures causing the reflection of the waves. If the measurement is done in close proximity to a strong reflection point, e.g. bifurcation, the measured PWV strongly deviates from the fundamental PWV. If the measurement is done further away from strong reflections, the deviation between the measured PWV and the fundamental PWV will be negligible. This is typically the case in long vessels like the aorta, where PWV is generally measured further away from points of strong reflections. Relatively short vessels, however, may present PWV values varying almost along the entire length of the vessel. Accordingly, it is beneficial to determine a fundamental PWV for the vessel, a value with minimum effect of reflection, which allows an improved assessment of the condition of the vessel, in particular for classification of the patient according to a predicted therapeutic benefit. In an embodiment of the apparatus, the limit value represents the most reflection-free pulse wave velocity value, ascertained by an asymptotic limit of the plurality of pulse wave velocity values along the length of the vessel. The PWV of a short homogenous vessel segment prior to a distal bifurcation is strongly influenced by wave reflection. The influence diminishes as the distance from the bifurcation increases. The limit value may be ascertained by calculation of an asymptotic limit of the plurality of pulse wave velocity values along the length of the vessel segment for which measurements are available. Based on the function of the pulse wave velocity values along the length of the vessel segment, a recommendation may be generated by the processor, and then output to a user interface, advising the user to continue PWV measurements at larger distances from the bifurcation, in order to further improve the accuracy of the fundamental PWV calculation.

In a further embodiment, the received plurality of measurements are related to a renal artery and the processor is configured to recommend the patient eligible for a renal denervation therapy based on the limit value compared to a predetermined threshold value. The main branches of the renal arteries are relatively short (typically 4-6 cm in length), and the measured PWV is influenced strongly by the reflections. Therefore, for most of the length of the renal artery, if not for the entire length, the PWV is substantially different than it would be without any reflections. Evidence based on clinical studies indicates that the predetermined threshold value for a successful renal denervation is a renal PWV of about 10 m/s. Patients for which the PWV in the renal artery is lower than the threshold value did not have sufficient benefit from a renal denervation (RDN) therapy, meaning that the resistant hypertension (high blood pressure not controlled by medication) did not sufficiently decrease or did even increase upon RDN therapy. The classification for eligibility of the patients for RDN therapy could be done based on a PWV measurement too close to a bifurcation, or based on a spatial average of multiple PWV values along the renal artery, in which cases the PWV value used for classification is probably an overestimation of the fundamental PWV value. Therefore, it could result in an incorrect eligibility classification of the patient for RDN treatment. A recommendation of the patient for eligibility for a renal denervation therapy based on the fundamental PWV has the advantage that a patient would not undergo an RDN treatment when the risks related to the treatment outweigh a predicted therapeutic benefit, despite the fact that a PWV value in a portion of the renal artery or a spatial average PWV value may exceed the threshold value. A tolerance margin of $\pm 10\%$ to the threshold value may be applicable to account for inter-patient variables, e.g. age, gender, medical condition history.

In a second aspect a system is provided for classification of the patient according to a predicted therapeutic benefit, comprising:

an apparatus of any of the embodiments according to the first aspect of the invention;
a measurement arrangement configured to provide the plurality of measurements along the length of the vessel of the patient;
a user interface configured to display the limit value of the pulse wave velocity values for classification of the patient according to the predicted therapeutic benefit.

In a first exemplary embodiment of the system, the measurement arrangement comprises:

an intravascular device having two sensors configured to measure physiological information within the vessel, wherein the intravascular device is adapted to provide measurements at a fixed location within the vessel with the first sensor and to provide measurements at various locations along the vessel with the second sensor. Optionally the measurement arrangement may comprise a control unit connected to the intravascular device, which is configured to adjust a distance between the two sensors.

In a second exemplary embodiment of the system, the measurement arrangement comprises:

two intravascular devices each having a sensor configured to measure physiological information within the vessel;
a control unit connected to the two intravascular devices, which is configured to move the two intravascular devices within the vessel in a synchronized manner.

Optionally, in the system according to the first and second exemplary embodiments, the measurement arrangement may further comprise an extracorporeal imaging apparatus configured to provide morphological information of the vessel, and the processor may be configured to co-register the morphological information of the vessel with temporal locations of the two sensors.

The physiological information in the first and second exemplary embodiment of the system is at least one of a pressure and a flow velocity of a medium in the vessel.

In a third exemplary embodiment of the system, the measurement arrangement comprises an intracorporeal imaging device configured to provide the plurality of measurements along the length of the vessel. The intracorporeal imaging device comprises at least one of an ultrasound sensor and an OCT sensor.

In a fourth exemplary embodiment of the system, the measurement arrangement comprises an extracorporeal imaging apparatus configured to:

provide morphological information of the vessel;
the plurality of measurements along the length of the vessel of the patient.

In a fifth exemplary embodiment of the system, the measurement arrangement comprises:

an intravascular device having a sensor configured to measure physiological information within the vessel, wherein the intravascular device is adapted to provide measurements at various locations along the vessel with the sensor; and an apparatus connectable to the patient and configured to provide electrical activity measurements of a heart of the patient.

In any of the exemplary embodiments of the systems, the extracorporeal imaging apparatus may use at least one of the following imaging modalities: cardiac CT angiography, MR angiography, ultrasound imaging. The morphological information of the vessel may comprise two- or three-dimensional (2D or 3D) data, from which 2D or 3D images of the vessel may be rendered.

In a third aspect of the invention a method of classification of a patient is presented, comprising:

receiving a plurality of measurements along a length of a vessel of a patient;
ascertaining a plurality of pulse wave velocity values along the length of the vessel based on the plurality of measurements;
ascertaining a limit value of the pulse wave velocity values for classification of the patient according to the predicted therapeutic benefit. The advantages of the method have been discussed with respect to the first aspect of the invention.

According to a further aspect, a computer program element is provided for controlling an apparatus according to the first aspect of the invention, which when being executed by a processor or a processing unit, is adapted to perform the method steps according to the third aspect of the invention. A computer readable medium having stored the program element thereon is provided.

These and other aspects of the invention will be apparent from and elucidated with reference to the embodiments described hereinafter.

BRIEF DESCRIPTION OF THE DRAWINGS

In the following drawings:

Fig. 1A shows simulation results of the PWV along a length of five homogeneous vessels (V1 to V5) with different mechanical properties that are connected to outlet vessels, which are impedance matched to avoid reflections.
Fig. 1B shows simulation results of the PWV along a length of five homogeneous vessels (V1 to V5) with different mechanical properties that are connected to outlet vessels, which are not impedance matched, therefore exhibiting reflections.
Fig. 2 is a schematic diagram illustrating an intravascular device positioned within the renal anatomy.
Fig. 3 is a diagrammatic schematic view of an exemplary system for ascertaining a fundamental PWV of a vessel.
Fig. 4 is an example of ascertaining delay from two measured pressure signals.
Fig. 5 is an example of piecewise linear regression used to approximate the location dependent delay ascertained according to Fig. 4 for pressure measurements along a vessel segment.
Fig. 6 represents the PWV values ascertained along the length of the vessel segment, together with the position of the wave reflector.
Fig. 7 exemplarily illustrates the calculation of the fundamental PWV.
Fig. 8 is a flowchart illustrating a method of calculating a fundamental pulse wave velocity of a vessel.

DETAILED DESCRIPTION OF EMBODIMENTS

Pulse wave velocity measurement is a well-established way to measure the compliance of major blood vessels. Especially, aortic and carotid PWV is used to evaluate the risk of cardiovascular events.

PWV is given by the Moens-Korteweg equation:

$$PWV = \sqrt{\frac{E \cdot h}{2r \cdot \rho}} \qquad\qquad (\text{Eq. 1})$$

where E is the Young's modulus, $r$ is the vessel radius, $h$ is the wall thickness, and $\rho$ the blood density. This "fundamental" PWV only depends on the mechanical properties of the vessel and the fluid.

There are multiple ways to determine PWV. The most common one is determining the delay in arrival time between the pulse from one measurement location to the next. This method works best for long vessels like the aorta, as the PWV value is relatively high in major arteries and therefore the distance should be large to accurately measure the time delay. The measured PWV value is then a single value, a spatial average PWV over a relatively long length.

The measured PWV can vary along the length of even a homogenous vessel due to the effect of wave reflections from bifurcations, stenoses or other structures with non-ideal impedance matching. These reflected waves will influence the measured PWV. Typically, these deviations from the fundamental PWV are small, but if the measurement is performed in close proximity to a strong reflection point, the measured PWV value can strongly deviate from the fundamental PWV given by Eq. 1. This is typically the case for short vessels like the renal arteries, where a strong reflection point (the bifurcation at the distal end of the artery) may inevitably be close to the measurement point.

The PWV measurement in renal arteries is used to determine whether a hypertension patient is likely to respond to renal denervation treatment. A PWV larger than approximately 10 m/s is an indication that a patient is likely to respond to RDN. As the main branch of the renal arteries are relatively short (typically 4-6 cm in length), the measured PWV will be influenced by the reflections, and therefore will artificially be different from what it would be without any reflections.

The pressure and flow pulses from the heart undergo various reflections at bifurcations and at locations where the vessel properties change (e.g. where a vessel is narrowing or where plaque changes the elasticity of the vessel wall). But it is typically not accounted for that these reflections change the PWV of the blood vessel proximal to the reflections.

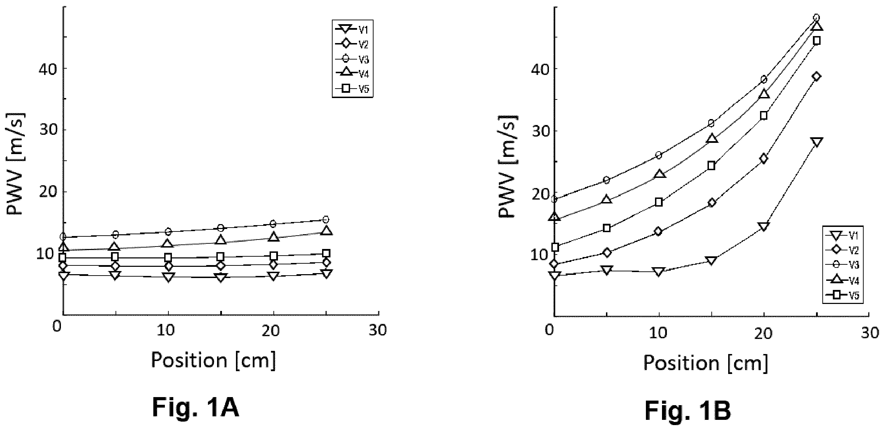

The effect is shown in Figs. 1A and IB. The graphs show the PWV along the length of five homogeneous vessels (V1 to V5) based on simulations, wherein the vessels have different mechanical properties. In Fig. 1A, the vessels VI to V5 are connected to impedance matched outlet vessels. As a result, there are no wave reflections and the PWVs are about constant along the length of the vessels. In Fig. 1B, the vessels are connected to outlet vessels, which are not impedance matched. This is what typically happens at the end of a vessel if there is a bifurcation or a stenosis. There are reflections at the end of the vessels, and the PWVs are changing along the length of the vessels, even though the vessels VI to V5 are still homogenous and their properties have not been altered between the two simulations. The only condition that has been modified is related to the properties of the simulated downstream vasculature.

This yield to the insights that the measured PWV can vary significantly along the length of a short blood vessel, in which case compliance of the vessel cannot be determined from a single PWV measurement, but should be determined from the underlying, fundamental PWV that would have been measured in the absence of the reflections. The reflection free, fundamental PWV can be determined from a spatially resolved series of PWV measurements along the length of vessel e.g. by determining an asymptotic limit value.

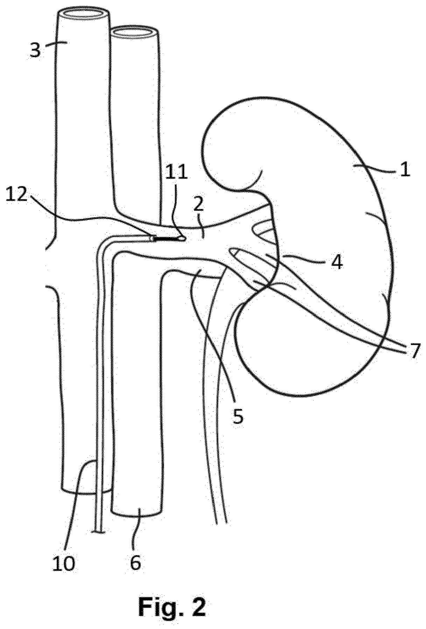

Fig. 2 illustrates an intravascular device 10 introduced within the human renal anatomy. The human renal anatomy includes kidneys 1 that are supplied with oxygenated blood by right and left renal arteries 2, which branch off an abdominal aorta 3 at the renal ostia to enter the hilum 4 of the kidney 1 (for simplicity reason only one side of the renal anatomy is illustrated). The abdominal aorta 3 connects the renal arteries 2 to the heart (not shown). Deoxygenated blood flows from the kidneys 1 to the heart via renal veins 5 and an inferior vena cava 6. Specifically, the flexible elongate member of the intravascular device 10 is shown extending through the abdominal aorta and into the left renal artery 2. In alternate embodiments, the intravascular device 10 may be sized and configured to travel through the inferior renal vessels 7 as well. Left and right renal plexi or nerves (not shown) surround the left and right renal arteries 2, respectively.

Anatomically, the renal nerve forms one or more plexi within the adventitial tissue surrounding the renal artery 2. For the purpose of this disclosure, the renal nerve is defined as any individual nerve or plexus of nerves and ganglia that conducts a nerve signal to and/or from the kidney 1 and is anatomically located within the adventitial tissue surrounding the renal artery 2, parts of the abdominal aorta 3 where the renal artery 2 branches off the aorta, and/or on inferior branches of the renal artery. Nerve fibers contributing to the plexi arise from the celiac ganglion, the lowest splanchnic nerve, the corticorenal ganglion, and the aortic plexus. The renal nerves extend in intimate association with the respective renal arteries into the substance of the respective kidneys 1. The nerves are distributed with branches of the renal artery to vessels of the kidney 1, the glomeruli, and the tubules. Each renal nerve generally enters each respective kidney 1 in the area of the hilum 4 of the kidney, but may enter the kidney 1 in any location, including the location where the renal artery 2 or a branch of the renal artery enters the kidney 1.

Proper renal function is essential to maintenance of cardiovascular homeostasis so as to avoid hypertensive conditions. Excretion of sodium is key to maintaining appropriate extracellular fluid volume and blood volume, and ultimately controlling the effects of these volumes on arterial pressure. Under steady-state conditions, arterial pressure rises to that pressure level which results in a balance between urinary output and water and sodium intake. If abnormal kidney function causes excessive renal sodium and water retention, as occurs with sympathetic overstimulation of the kidneys through the renal nerves, arterial pressure will increase to a level to maintain sodium output equal to intake. In hypertensive patients, the balance between sodium intake and output is achieved at the expense of an elevated arterial pressure in part as a result of the sympathetic stimulation of the kidneys through the renal nerves. Renal denervation may help alleviate the symptoms and sequelae of hypertension by blocking or suppressing the efferent and afferent sympathetic activity of the kidneys 1.

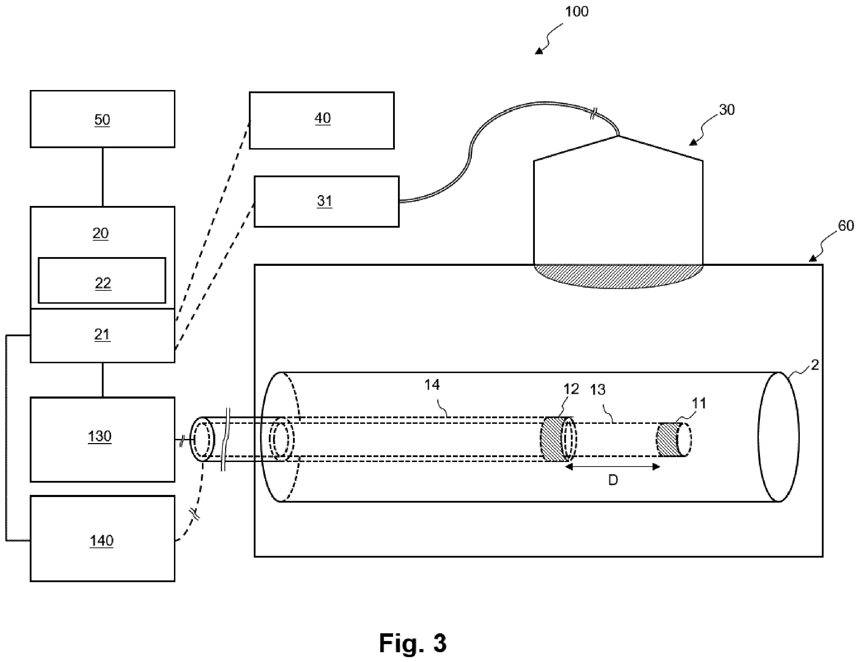

Systems for classification of the patient according to a predicted therapeutic benefit are described with reference to Fig. 3. In various exemplary embodiments, some of the elements of the system may be missing, or they may be substituted with alternative elements with similar function. The vessel 2 may be right or left renal artery 2 described with reference to Fig. 2, which is distally branching off into the inferior renal vessels 7.

In a first exemplary embodiment, the system comprises a measurement arrangement wherein an intravascular device 10 consists of a guidewire 13 (e.g. a Philips Volcano Verrata or Varsity pressure wire) and a guiding catheter 14. The guidewire, comprising a first pressure sensor 11 located at its distal portion, is connected to a patient interface module (PIM) 130 configured to control the motion of the device along the vessel 2 during measurements (mechanical pullback). A second pressure sensor 12, located on the guiding catheter 14, is stationary placed nearby. The PIM 130 controls the mechanical pullback of the guidewire and transmits the pressure signal from the first sensor to the console, which is a processing apparatus 20, comprising an input unit 21 and a processor 22. The pressure measurement signal from the second sensor 12 may directly be transmitted to the input unit 21, through the PIM 130 or a second PIM 140. The pressure signals may be transmitted through wired or wireless connection either directly from the intravascular device 10 to the processing apparatus 20, or from the PIM(s) 130,140 to the processing apparatus. The first pressure sensor 11 is placed distal to the second pressure sensor 12 at various distances D in the vessel, and measurement data is collected successively for changing the distance between the two sensors by mechanical pull-back of the guidewire controlled by the PIM 130. Once the mechanical pullback is activated, the guidewire is slowly pulled back (for example at a constant speed of 1 mm/s) while the processing apparatus simultaneously records both pressure signals with a high sampling rate (for example 10 kHz) and registers the relative locations of the two sensors with the pressure measurement data.

In alternative embodiment, the sensing location of the second sensor 12 may be in the vessel (e.g. aorta) that supplies blood to the vessel (e.g. renal artery) in which the successive measurement locations of sensor 11 are. This is advantageous for short vessels, as only one intravascular device 13 needs to be introduced within the short vessel, the second intravascular device 14 not influencing further the blood flow within the short vessel. A further option is to configure one or both intravascular devices such that the pressure sensor(s) themselves are located outside of the body of the subject, with the lumen(s) within the intravascular device(es) simply transducing the hemostatic pressure.

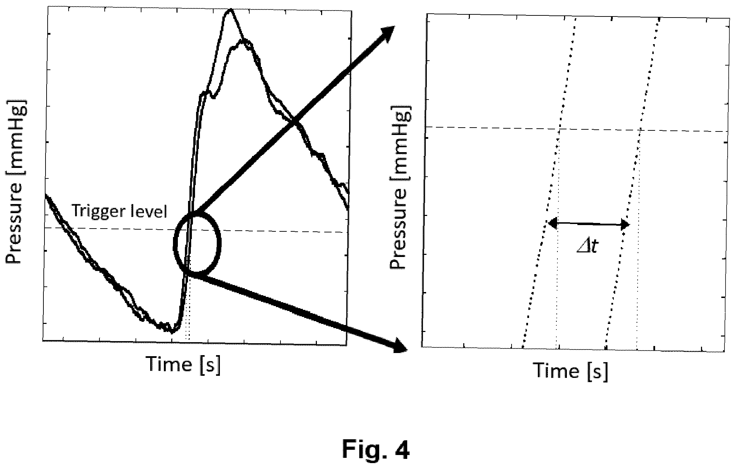

Due to the fact that the pressure sensors 11 and 12 (or the openings of the lumens of the intravascular devices connecting to the pressure sensors located outside the body of the subject) are at different locations in the blood stream, the pressure signals from sensors 11 and 12 will show a position-dependent time delay $\Delta t(x)$. Fig. 4 shows typical pressure signals (pressure versus time over one heartbeat). The two curves represent the measured signals with stationary pressure sensor 12 and the moving pressure sensor 11, respectively. Because the pressure sensors are at different locations, the pressure signals will be somewhat different and there will be a delay in the rise of the pressure signal. The graphs show one way to extract this delay $\Delta t$ from the signal, by defining a trigger level and timing when the signals exceed the trigger level. There are alternative ways to extract the delay, which are well-known in the art. A value $\Delta t(x)$ is determined for each heart beat during the pullback.

If the PWV were constant, then the relationship between the delay $\Delta t$ and the position $x$ would be linear:

$$\Delta t(x) = m\,x + b \qquad\qquad (\text{Eq. 2})$$

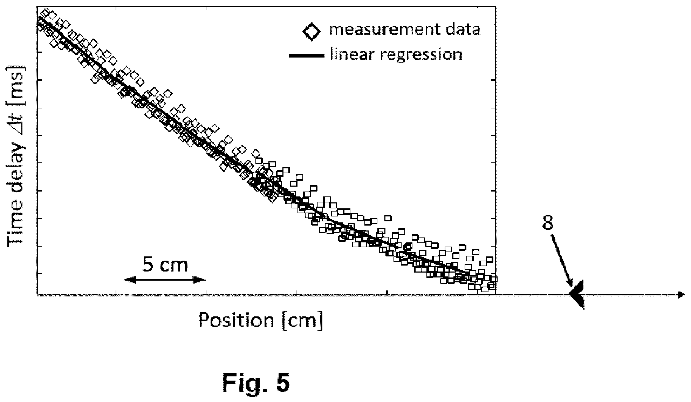

with the slope m and the intercept b. However, even if the PWV is not constant, if the pullback speed is slow enough, then one can find pieces of the graph that are approximately linear. Fig. 5 shows how piecewise linear regression can be used to approximate the measured data and determine the local slope m(x). The continuous line segments are piecewise linear regressions. In this example, there was a strong reflection point 8 at 3.5 cm from the most distal measurement point. From the local linear slope m(x) one can determine PWV via:

$$PWV(x) = \frac{1}{m(x)} \qquad \text{(Eq. 3)}$$

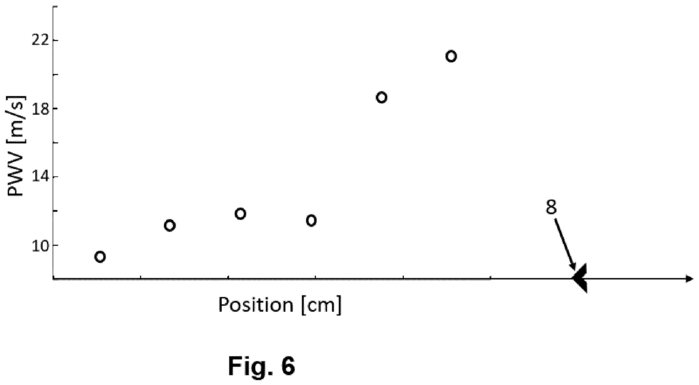

The PWV values along the length of the vessel segment, calculated by using Eq. 3, is presented in Fig. 6, together with the position of the strong wave reflector (e.g. a bifurcation).

The processing apparatus 20 determines the location dependent PWV along the length of the vessel $V$(x), and it may output the graph to a display 50 for assessment by a physician.

The processing apparatus may further derive and output to the display additional information such as interpolation and extrapolation graphs, and estimated fundamental PWV value ($PWV_f$) from the extrapolation. The function can be, for example, described by an exponential curve:

$$PWV = a \, e^{b \, (x-x0)} + PWV_f \qquad \text{(Eq. 4)}$$

wherein x is the location along the vessel segment, PWV is the measured PWV, $PWV_f$ is the fundamental or most reflection free PWV and *a, b, x0* are parameters that determine the slope of the curve. It is important to note that it is not necessary to know the distance from the strong reflection, nor is necessary to be a single strong reflection (instead there can be multiple medium to strong reflection points adding up).

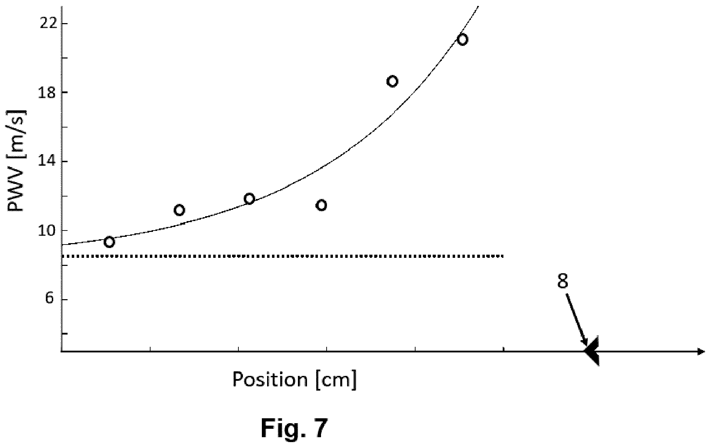

Fig. 7 exemplarily illustrates the calculation of the fundamental PWV, wherein the circles represent the measured location dependent PWV along the vessel segment, with other words the PWV directly obtained by pressure measurements along the vessel segment. The solid line is an exponential fit to the data, according to Eq. 4. The dotted black line is the best estimate of the fundamental pulse wave velocity $PWV_f$.

In an alternative of the first exemplary embodiment of the system, the pressure sensor 12 on the guiding catheter may be placed stationary in the aorta 3, while the pressure sensor 11 on the guidewire may be mechanically pulled back by the PIM 130 along a segment of the renal artery. The signal transmission and processing for obtaining the fundamental PWV of the renal artery is identical to the description relating to Figs. 3 to 7.

In the second exemplary embodiment of the system, two pressure sensors are pulled back in parallel. This is easily done by using a guidewire with two pressure sensors, but it can also be done with two single-sensor pressure-wires by clamping the wires into the same PIM 130 for mechanical pullback. In both cases, the distance D between the sensors is known.

The mechanical pullback of the intravascular devices occurs slowly, with both sensors in parallel, while the processing apparatus simultaneously records both pressure signals with a high sampling rate. As described above, the local time delay $\Delta t$(x) between the pressure signals from both sensors is calculated for each heart beat. The position x is known from the pullback speed. The local PWV is then calculated by using:

$$PWV(x) = \frac{D}{\Delta t(x)}. \qquad \text{(Eq. 5)}$$

Subsequently, the fundamental PWV is determined by using Eq. 4.

Optionally, in any of the systems according to the first and second exemplary embodiments, the measurement arrangement may further comprise an extracorporeal imaging apparatus 30 configured to provide morphological information of the vessel inside the body 60 of the patient, and the processor may be configured to co-register the morphological information of the vessel with temporal locations of the two sensors. The pullback speed of the sensors can be determined via co-registration with the imaging modality, e.g. by visualization of the locations of the sensors by the respective imaging modality. This enables a manual pull-back by the user, instead of a mechanical pull-back controlled by the PIM. The extracorporeal imaging apparatus may use at least one of the following imaging modalities: cardiac CT angiography, MR angiography, ultrasound imaging. The morphological information of the vessel may comprise two- or three-dimensional (2D or 3D) data, from which 2D or 3D images of the vessel may be rendered. Specific markers for the listed imaging modalities to locate the position of the sensor in the vessel are known in the art, more specifically echogenic markers for ultrasound imaging, radiopaque markers for x-ray based imaging and markers for magnetic resonance imaging.

Instead of using only pressure sensors for measurement of physiological information in the first and second exemplary embodiments of the system, an alternative option is to use an intravascular device with a combined flow and pressure sensor (e.g. a Philips Volcano Combowire). In this case, one does not need a second reference sensor. The flow-pressure sensor will measure the local pressure $p(x, t)$ and the flow velocity $u(x, t)$ while being slowly pulled back. There are multiple ways to derive the local PWV from these measurements. For example one can use the following equation:

$$PWV(x) = \frac{1}{\rho} \sqrt{\frac{\sum dp^2}{\sum du^2}}, \qquad\qquad (\text{Eq. } 6)$$

where $dp$ and $du$ are the temporal changes in pressure and flow from one measurement point to the next and $\rho$ is the density of the liquid (typically blood). The summations are over each heart cycle.

An alternative method in determining the PWV is the slope for the reflection-free part of the p-u curve. The PWV is then:

$$PWV(x) = \frac{1}{\rho} \frac{dp}{du} \qquad\qquad (\text{Eq. } 7)$$

Subsequently, the fundamental PWV is determined by using Eq. 4.

In a further alternative for the first and second exemplary embodiments of the system, an intravascular device with flow sensors is used, instead of pressure sensors. From the measurement information it is determined the time delay that a modification of a flow velocity takes to propagate from the first sensor to the second sensor, wherein the modification of the flow velocity is caused by the heartbeat, creating pulsatile blood motion.

In a third exemplary embodiment of the system, the intravascular device is an intracorporeal imaging device, configured to provide intravascular ultrasound (IVUS) or optical coherence tomography (OCT) measurements along the length of the vessel. The intracorporeal imaging device may comprise a first imaging element 11 and a second imaging element 12 coupled to the distal portion, wherein the first imaging element is configured to monitor a measurement value within the vessel at a first location, and wherein the second imaging element is configured to monitor the measurement value within the vessel at a second location spaced apart from the first location. The pulse wave velocity is determined from the time delay that it takes a change in the diameter of the vessel, a change of the distance to a wall of the vessel, or a change in the thickness of the vessel wall to be measured by the first and second imaging elements.

In a fourth exemplary embodiment of the system, the measurement arrangement comprises an extracorporeal imaging apparatus 30 configured to provide the morphological information of the vessel and the plurality of measurements along the length of the vessel of the patient. The extracorporeal imaging apparatus 30 may directly provide the measurement data to the processing apparatus 20, or it may have a control unit 31 for processing the morphological information. The control unit 31 may have various functions, depending on the imaging modality, e.g. beam forming for ultrasound imaging with extracorporeal ultrasound probe, wherein imaging of the vessel morphology can be captured in a single large aperture, together with the propagation of a change of the vessel diameter or a vessel wall thickness, from which the PWV is determined. If the extracorporeal imaging apparatus 30 is suitable for any of the CT angiography and MR angiography, then the control unit 31 may control dosing contrast medium for the respective imaging modality, provide segmentation of the imaged anatomy, and calculate propagation of a change of flow and/or vessel morphology, based on which PWV is determined by the processing apparatus 20.

In a fifth exemplary embodiment of the system, the measurement arrangement comprises an intravascular device having a sensor configured to measure physiological information within the vessel, being flow velocity and/or pressure, wherein the intravascular device is adapted to provide measurements at various locations along the vessel with the sensor by pull-back controlled by the PIM 130, and further comprising an apparatus connectable to the patient and configured to provide electrical activity measurements of the heart of the patient, e.g. ECG device 40. Contrary to the first exemplary embodiment of the system, the fifth exemplary embodiment of the system does not need to use a stationary pressure or flow sensor. Instead, it uses measurement of ECG (electrocardiogram) signal originating at the body of the patient. The system derives a trigger signal from the rise in the ECG signal. The time delay $\Delta t$ between the ECG signal and the physiological signal measured by the sensor at various locations along the segment of the vessel, is derived from the difference in the timing between the two signals. The PWV and the fundamental PWV is determined in a similar way as in the first exemplary embodiment of the system.

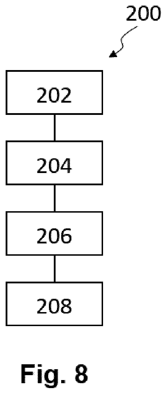

Fig. 8 is a flowchart illustrating a method 200 of classification of a patient according to the predicted therapeutic benefit. In step 202 the processing apparatus receives form a measurement arrangement a plurality of measurements along a length of a vessel of a patient. The measurement arrangement may be any of the exemplary embodiments of the system or their alternatives. In step 204 the processing apparatus 20 ascertains a plurality of pulse wave velocity values along the length of the vessel based on the plurality of measurements. In step 206 the processing apparatus 20 ascertains a limit value of the pulse wave velocity values for classification of the patient according to the predicted therapeutic benefit. The limit value represents the most reflection-free pulse wave velocity value, which can be for example ascertained by an asymptotic limit of the plurality of pulse wave velocity values along the length of the vessel. Optionally, in step 208 the processing apparatus may recommend the patient eligible for a therapy based on the limit value compared to a predetermined threshold value.

A computer program element can be provided for controlling the processing apparatus 20, which when being executed by a processor 22 or a processing unit, is adapted to perform the steps according to the method 200. A computer readable medium having stored the program element thereon can also be provided.

Although the methodology and systems for classification of the patient according to a predicted therapeutic benefit are described with reference to renal arteries and corresponding renal denervation treatment, the invention is not limited to the exemplarily described clinical application, but it is applicable to any assessment of vessel condition where the corresponding distal vessel network cause wave reflections leading to variation of the PWV values along the vessel (e.g. coronary vasculature, cerebral vasculature, hepatic vasculature). Assessment of the condition of the vessel and/or classification of the patients according to a predicted therapeutic benefit based on single PWV value may be prejudiced by the proximity of strong reflection sources (e.g. bifurcations, stenosis). The fundamental PWV provides a solution to improve assessment of the condition of the vessel and/or classification of the patients according to a predicted therapeutic benefit.

Persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

**Claims**

1.  An apparatus (20) comprising:

    an input unit (21) configured to receive a plurality of measurements along a length of a vessel (2) of a patient;
    a processor (22) configured to:

    ascertain a plurality of pulse wave velocity values along the length of the vessel based on the plurality of measurements;
    ascertain a limit value of the pulse wave velocity values for classification of the patient according to a predicted therapeutic benefit.

2.  The apparatus of claim 1, wherein the limit value represents the reflection-free pulse wave velocity value, ascertained by an asymptotic limit of the plurality of pulse wave velocity values along the length of the vessel.

3.  The apparatus of claim 1 or 2, wherein the received plurality of measurements are related to a renal artery (2) and the processor is configured to recommend the patient eligible for a renal denervation therapy based on the limit value compared to a predetermined threshold value.

4.  The apparatus of claim 3, wherein the predetermined threshold value is about 10 m/s.

5.  A system (100) for classification of the patient according to a predicted therapeutic benefit, comprising:

    an apparatus (20) according to any of the preceding claims;
    a measurement arrangement configured to provide the plurality of measurements along the length of the vessel (2) of the patient;
    a user interface (50) configured to display the limit value of the pulse wave velocity values for classification of the patient according to the predicted therapeutic benefit.

6.  The system of claim 5, wherein the measurement arrangement comprises:

    an intravascular device (10) having two sensors (11,12) configured to measure physiological information within the vessel, wherein the intravascular device is adapted to provide measurements at a fixed location within the vessel with the first sensor (12) and to provide measurements at various locations along the vessel with the second sensor (11).

7. The system of claim 6, wherein the measurement arrangement comprises a control unit (130) connected to the intravascular device (10), which is configured to adjust a distance between the two sensors (11,12).

8. The system of claim 5, wherein the measurement arrangement comprises:

   two intravascular devices (13,14) each having a sensor (11,12) configured to measure physiological information within the vessel;
   at least a control unit (130,140) connected to the two intravascular devices, which is configured to move the two intravascular devices within the vessel in a synchronized manner.

9. The system of any of the claims 6 to 8,
   wherein the measurement arrangement further comprises an extracorporeal imaging apparatus (30) configured to provide morphological information of the vessel;
   wherein the processor (22) is configured to co-register the morphological information of the vessel with temporal locations of the two sensors (11,12) within the vessel.

10. The system of any of the claims 6 to 9, wherein the physiological information is at least one of a pressure and a flow velocity of a medium in the vessel.

11. The system of claim 5, wherein the measurement arrangement comprises an intracorporeal imaging device (10) configured to provide the plurality of measurements along the length of the vessel.

12. The system of claim 11, wherein the intracorporeal imaging device comprises at least one of an ultrasound sensor and an OCT sensor.

13. The system of claim 5, wherein the measurement arrangement comprises an extracorporeal imaging apparatus (30) configured to:

   provide morphological information of the vessel;
   the plurality of measurements along the length of the vessel of the patient.

14. The system of claim 5, wherein the measurement arrangement comprises:

   an intravascular device (10) having a sensor (11) configured to measure physiological information within the vessel, wherein the intravascular device is adapted to provide measurements at various locations along the vessel with the sensor;
   and an apparatus (40) connectable to the patient and configured to provide electrical activity measurements of a heart of the patient.

15. A method (202) of classification of a patient according to a predicted therapeutic benefit, comprising:

   receiving (202) a plurality of measurements along a length of a vessel of a patient;
   ascertaining (204) a plurality of pulse wave velocity values along the length of the vessel based on the plurality of measurements;
   ascertaining (206) a limit value of the pulse wave velocity values for classification of the patient according to the predicted therapeutic benefit.

Fig. 1B

Fig. 1A

**Fig. 2**

Fig. 3

Fig. 4

**Fig. 5**

Fig. 6

**Fig. 7**

200

202

204

206

208

**Fig. 8**

Europäisches
Patentamt

European
Patent Office

Office européen
des brevets

# EUROPEAN SEARCH REPORT

Application Number

EP 18 20 2488

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/34724 A2 (FLORENCE MEDICAL LTD [IL]; DGANY ELHANAN [IL] ET AL.) 15 July 1999 (1999-07-15) * column 6, lines 17-30; figure 1 * * page 40, line 23 - page 44, line 9 * * page 34, line 23 - page 37, line 11 * * page 31, lines 16-26 * | 1-15 | INV. A61B8/06 A61B8/12 A61B5/0215 A61B5/0285 A61B5/02 A61B5/00 |
| X | WO 2017/198867 A1 (KONINKLIJKE PHILIPS NV [NL]) 23 November 2017 (2017-11-23) * page 12, line 24 - page 16, line 15 * * page 19, lines 7-21; figure 2 * * page 25, lines 9-28; figure 8 * | 1,6,8,9, 11-13 | ADD. A61B5/026 A61B5/0456 |
| A,D | US 2010/113949 A1 (SATHYANARAYANA SHASHIDHAR [US]) 6 May 2010 (2010-05-06) * paragraphs [0042] - [0044] * | 6,15 | |
| A | US 6 331 162 B1 (MITCHELL GARY F [US]) 18 December 2001 (2001-12-18) * column 5, line 65 - column 6, line 22 * | 1-15 | |

TECHNICAL FIELDS
SEARCHED      (IPC)

A61B

The present search report has been drawn up for all claims

| Place of search | Date of completion of the search | Examiner |
|---|---|---|
| The Hague | 2 April 2019 | Loveniers, Kris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................
& : member of the same patent family, corresponding document

EPO FORM 1503 03.82 (P04C01)

**EP 3 643 244 A1**

## ANNEX TO THE EUROPEAN SEARCH REPORT
## ON EUROPEAN PATENT APPLICATION NO.

EP 18 20 2488

02-04-2019

| Patent document cited in search report | | | Publication date | Patent family member(s) | | | Publication date |
|---|---|---|---|---|---|---|---|
| WO 9934724 | A2 | | 15-07-1999 | AU | 1781699 | A | 26-07-1999 |
| | | | | WO | 9934724 | A2 | 15-07-1999 |
| WO 2017198867 | A1 | | 23-11-2017 | CN | 109152533 | A | 04-01-2019 |
| | | | | EP | 3457911 | A1 | 27-03-2019 |
| | | | | WO | 2017198867 | A1 | 23-11-2017 |
| US 2010113949 | A1 | | 06-05-2010 | US | 2006058653 | A1 | 16-03-2006 |
| | | | | US | 2010113949 | A1 | 06-05-2010 |
| | | | | WO | 2006023924 | A1 | 02-03-2006 |
| US 6331162 | B1 | | 18-12-2001 | NONE | | | |

EPO FORM P0459

For more details about this annex : see Official Journal of the European Patent Office, No. 12/82

**REFERENCES CITED IN THE DESCRIPTION**

**Patent documents cited in the description**

*   US 20100113949 A1

**Non-patent literature cited in the description**

*   **FINEGOLD et al.** Systematic evaluation of haemodynamic parameters to predict haemodynamic responders to renal artery denervation, EuroInterven-tion. *Abstracts EuroPCR,* 2016